(19) Europäisches Patentamt / European Patent Office / Office européen des brevets

(11) **EP 3 761 857 B1**

(12) **FASCICULE DE BREVET EUROPEEN**

(45) Date de publication et mention de la délivrance du brevet:
**07.06.2023 Bulletin 2023/23**

(21) Numéro de dépôt: **19714996.6**

(22) Date de dépôt: **11.03.2019**

(51) Classification Internationale des Brevets (IPC):
**A61B 5/00** *(2006.01)*    **G02F 1/11** *(2006.01)*
**G01N 21/17** *(2006.01)*    **G01N 21/49** *(2006.01)*
**G01N 29/24** *(2006.01)*

(52) Classification Coopérative des Brevets (CPC):
**A61B 5/7257; A61B 5/0097; A61B 5/7228;**
**G01N 21/1702; G01N 21/1717; G01N 21/49;**
**G01N 29/2418; G02F 1/11;** G01N 2021/1787

(86) Numéro de dépôt international:
**PCT/EP2019/055990**

(87) Numéro de publication internationale:
**WO 2019/170907 (12.09.2019 Gazette 2019/37)**

(54) **PROCÉDÉS ET SYSTÈMES D'IMAGERIE ACOUSTO-OPTIQUE**

AKUSTISCH-OPTISCHE BILDGEBUNGSVERFAHREN UND SYSTEME

ACOUSTO-OPTIC IMAGING METHODS AND SYSTEMS

(84) Etats contractants désignés:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priorité: **09.03.2018 FR 1852081**

(43) Date de publication de la demande:
**13.01.2021 Bulletin 2021/02**

(73) Titulaires:
- **Centre national de la recherche scientifique**
  **75016 Paris (FR)**
- **Ecole Supérieure de Physique et de Chimie**
  **Industrielles de la Ville de Paris**
  **75005 Paris (FR)**
- **SORBONNE UNIVERSITE**
  **75006 Paris (FR)**
- **Univ Paris XIII Paris-Nord Villetaneuse**
  **93430 Villetaneuse (FR)**

(72) Inventeurs:
- **RAMAZ, François**
  **77120 Coulommiers (FR)**
- **GENNISSON, Jean-Luc**
  **95000 Cergy (FR)**
- **LAUDEREAU, Jean-Baptiste**
  **75015 Paris (FR)**
- **DUPUY, Clément**
  **94400 Vitry sur Seine (FR)**
- **TUALLE, Jean-Michel**
  **92160 Antony (FR)**

(74) Mandataire: **Osha Liang**
  **2, rue de la Paix**
  **75002 Paris (FR)**

(56) Documents cités:
WO-A1-98/50781          WO-A1-2016/193554
US-A1- 2003 030 886     US-A1- 2008 296 514

- CHIAO R Y ET AL: "Sparse array imaging with spatially-encoded transmits", ULTRASONICS SYMPOSIUM, 1997. PROCEEDINGS., 1997 IEEE TORONTO, ONT., CANADA 5-8 OCT. 1997, NEW YORK, NY, USA,IEEE, US, vol. 2, 5 octobre 1997 (1997-10-05), pages 1679-1682, XP010271620, DOI: 10.1109/ULTSYM.1997.663318 ISBN: 978-0-7803-4153-1

EP 3 761 857 B1

**Description**

DOMAINE

La présente description est relative aux procédés et aux systèmes d'imagerie acousto-optique.

Plus particulièrement, l'invention se rapporte notamment à un procédé d'imagerie acousto-optique pour imager une zone d'observation d'un milieu. Un tel procédé vise à obtenir, de manière non-invasive, une information sur les propriétés optiques d'une zone d'observation située en profondeur dans un milieu, par exemple des tissus biologiques. Les propriétés optiques peuvent être par exemple une couleur, une absorption, ou encore une structure des tissus biologiques de la zone d'observation. La zone d'observation est par exemple située à quelques millimètres ou centimètres en profondeur dans un objet, par exemple à l'intérieur d'un corps, d'un organe ou d'un objet.

ART ANTERIEUR

On connaît de tels procédés, dans lesquels on génère, dans la zone d'observation du milieu, une onde acoustique ultrasonore focalisée sur une tache focale dans la zone d'observation et on émet simultanément une onde lumineuse dans cette même zone. On obtient alors une information en détectant un signal lié au couplage entre l'onde lumineuse et la vibration acoustique dans le milieu. En effet, lorsqu'une onde ultrasonore, de fréquence acoustique $fa$ traverse un milieu diffusant (par exemple un tissu biologique ou autre), elle provoque un déplacement périodique des diffuseurs et une modulation périodique de l'indice de réfraction du milieu. Si une onde lumineuse incidente, notamment une onde laser, de fréquence incidente $fi$ est diffusée par le milieu, le mouvement des diffuseurs et la modulation de l'indice de réfraction du milieu génèrent une onde lumineuse marquée comprenant d'une part une composante porteuse à la fréquence incidente $fi$, et d'autre part une composante acousto-optique diffusée sur l'une ou l'autre des bandes latérales acoustiques, de fréquences $fao = fa \pm n * fi$.

De tels procédés sont notamment décrits dans « Ultrasound-mediated optical tomography: a review of current methods » de Daniel S. Elson, Rui Li, Christopher Dunsby, Robert Eckersley et Meng-Xing Tang, publié dans Interface Focus (2011) vol. 1, pages 632-648.

Ces procédés connus étaient lents du fait qu'il était nécessaire de balayer la zone d'observation avec une succession d'ondes ultrasonores focalisées. La réalisation d'une image impliquait d'émettre environ 200 000 ondes ultrasonores focalisées.

Un progrès considérable a déjà été apporté à ces procédés connus par l'invention décrite dans le document WO2016193554, qui prévoit d'envoyer dans la zone d'observation non pas des ondes ultrasonores focalisées, mais une succession d'ondes ultrasonores non-focalisées ayant des directions de propagation différentes. Cette méthode permet de fortement limiter le nombre tirs d'ondes ultrasonores nécessaires pour réaliser une image, par rapport à la méthode classique susmentionnée. En pratique, on peut ainsi aller jusqu'à diviser par 50 le nombre de tirs d'ondes ultrasonores pour réaliser une image.

RESUME

Il est apparu nécessaire de perfectionner encore les procédés et dispositifs connus d'imagerie acousto-optique, notamment pour améliorer leur résolution latérale (c'est-à-dire dans une direction parallèle au réseau de transducteurs ultrasonores) sans complexifier le dispositif ni perdre sensiblement en rapidité.

A cet effet, il est proposé un procédé d'imagerie acousto-optique pour imager une zone d'observation d'un milieu, le procédé comprenant

- une étape de mesure au cours de laquelle on acquière par voie optique une pluralité de signaux de mesure $S_{mj}(t)$ associés à des ondes acoustiques non-focalisées se propageant respectivement selon des directions de propagation m différentes, lesdites ondes acoustiques non focalisées étant émises dans la zone d'observation respectivement par un réseau de transducteurs ultrasonores régulièrement distribués spatialement, lesdites ondes acoustiques non focalisées étant émises, dans chaque direction de propagation m, successivement J fois avec J modulations spatiales périodiques j d'amplitude pour former des ondes acoustiques non focalisées modulées spatialement, les modulations spatiales périodiques j d'amplitude ayant une période spatiale identique selon au moins une direction de périodicité spatiale, et correspondant à un nombre donné P de transducteurs, les modulations spatiales périodiques j d'amplitude étant décalées spatialement en phase deux à deux, l'étape de mesure comportant, pour chaque modulation spatiale j d'amplitude, une pluralité d'opérations de mesure successives comprenant chacune les sous-étapes suivantes :

  o une sous-étape d'émission acoustique dans laquelle on fait émettre une onde acoustique non-focalisée modulée spatialement avec ladite modulation spatiale périodique j d'amplitude et se propageant selon une direction de propagation m,

o une sous-étape d'émission lumineuse dans laquelle on émet dans la zone d'observation, en même temps que l'onde acoustique non-focalisée modulée spatialement, une onde lumineuse incidente pour générer une onde lumineuse marquée modulée spatialement, comportant au moins une composante acousto-optique décalée en fréquence respectivement par l'onde acoustique non-focalisée modulée spatialement,

o une sous-étape d'acquisition dans laquelle on capte l'onde lumineuse marquée modulée spatialement et on acquiert ainsi le signal de mesure $S_{mj}(t)$ correspondant à la modulation spatiale périodique j d'amplitude et à l'onde acoustique non-focalisée se propageant dans la direction m,

- une étape de démodulation spatiale, au cours de laquelle on combine les différents signaux de mesure $S_{mj}(t)$ correspondant aux J modulations spatiales périodiques d'amplitude pour une même direction de propagation m, pour obtenir un signal démodulé $S_m(t)$ propre à la direction de propagation m,

- une étape de traitement au cours de laquelle on détermine une image d'au moins une partie de la zone d'observation à partir des signaux démodulés $S_m(t)$.

Grâce à ces dispositions, la modulation spatiale des ultrasons permet d'obtenir, par le signal acousto-optique, de l'information sur des fréquences spatiales plus élevées et donc d'améliorer la résolution latérale de l'image obtenue.

Le procédé d'imagerie acousto-optique peut en outre inclure l'une et / ou l'autre des caractéristiques suivantes :

- le réseau de transducteurs est linéaire et s'étend selon ladite direction de périodicité spatiale ;

- lesdites modulations spatiales périodiques j d'amplitude sont des fonctions binaires, de sorte que seuls certains transducteurs ultrasonores sont activés pour une modulation spatiale périodique j d'amplitude donnée ; les transducteurs ultrasonores sont ainsi activés selon un motif j périodique, de période P, et les modulations spatiales périodiques j d'amplitude décalées spatialement deux à deux d'une fraction de période spatiale;

- J est égal à 4, la période spatiale correspond à un nombre P de transducteurs multiple de 4 et les modulations spatiales périodiques d'amplitude sont décalées spatialement en phase deux à deux d'une phase correspondant au quart de ladite période spatiale;

- Au cours de l'étape de démodulation spatiale on calcule les transformées de Fourier temporelles $\check{S}_{mj}(\nu)$ des signaux de mesure $S_{mj}(t)$, $\nu$ étant la fréquence temporelle ; on détermine un signal $\check{S}_m(\nu)$ par une combinaison linéaire desdites transformées de Fourier temporelles $\check{S}_{mj}(\nu)$ ; et on détermine le signal démodulé $S_m(t)$ par transformée de Fourier inverse du signal $\check{S}_m(\nu)$.

- au cours de l'étape de démodulation spatiale, on calcule le signal démodulé $S_m(t)$ par transformée de Fourier inverse d'un signal $\check{S}_m(\nu)$, avec :

$$\tilde{S}_m(\nu) = \tilde{S}_{0m0}(\nu) + \tilde{S}_{1m0}(\nu) + \tilde{S}_{-1m0}(\nu) \qquad (17),$$

où :

$$\tilde{S}_{0m0}(\nu) = 1/4\big[\tilde{S}_{m0}(\nu) + \tilde{S}_{m1}(\nu) + \tilde{S}_{m2}(\nu) + \tilde{S}_{m3}(\nu)\big] \qquad (14)$$

$$\tilde{S}_{1m0}(\nu) = 1/4\big[\tilde{S}_{m0}(\nu) - \tilde{S}_{m2}(\nu) - i(\tilde{S}_{m1}(\nu) - \tilde{S}_{m3}(\nu))\big] \qquad (15)$$

$$\tilde{S}_{-1m0}(\nu) = 1/4\big[\tilde{S}_{m0}(\nu) - \tilde{S}_{m2}(\nu) + i(\tilde{S}_{m1}(\nu) - \tilde{S}_{m3}(\nu))\big] \qquad (16)$$

les termes $\check{S}_{mj}(\nu)$ sont les transformées de Fourier temporelles des signaux de mesure $S_{mj}(t)$,

j est un entier compris entre 0 et 3,

$\nu$ est la fréquence temporelle ;

- l'onde acoustique non-focalisée est choisie parmi une onde acoustique plane et une onde acoustique divergente ;

- les directions de propagation m des ondes acoustiques non-focalisées couvrent un secteur angulaire d'angle compris entre 30 et 50 degrés ;

- les directions de propagation m des ondes acoustiques non-focalisées sont séparées par un pas angulaire compris entre 0.5 degré et 2 degrés ;

- chaque opération de mesure est réitérée L fois pour acquérir L signaux de mesure bruts $S_{mjl}(t)$ associés à chaque direction de propagation m d'une onde acoustique non-focalisée et à chaque modulation spatiale périodique j d'amplitude, et lesdits L signaux de mesure $S_{mjl}(t)$ sont moyennés ensemble pour déterminer le signal de mesure $S_{mj}(t)$ utilisé pour l'étape de démodulation spatiale ;
- chaque signal de mesure $S_{mj}(t)$ est échantillonné à une fréquence supérieure à 2 mégahertz, de préférence supérieure à dix mégahertz ;
- l'étape de traitement comprend la mise en oeuvre d'une transformation de Radon inverse ;
- l'étape de traitement comprend la mise en oeuvre d'un algorithme de formation de voies ;
- l'étape de traitement comprend la mise en oeuvre d'un algorithme de rétroprojection ou de rétroprojection filtrée ;
- l'étape de traitement comprend les opérations de

  - déterminer une pluralité de tranches de profil associée à au moins un signal de mesure, chaque tranche de profil étant fonction d'une transformation de Fourier unidimensionnelle d'un signal de mesure associé,
  - déterminer un spectre bidimensionnel à partir de la pluralité de tranches de profil, et
  - déterminer au moins une valeur représentative d'une intensité lumineuse dans la zone d'observation, ladite valeur représentative étant fonction d'une transformation de Fourier bidimensionnelle inverse du spectre bidimensionnel ;

- on détermine le spectre bidimensionnel par recalage dans un espace de Fourier de la pluralité de tranches de profil, de préférence par recalage de chaque tranche de profil en fonction d'une direction de propagation d'une onde acoustique non-focalisée associée au signal de mesure associé à la tranche de profil.

Il est également proposé un système d'imagerie acousto-optique pour imager une zone d'observation d'un milieu, le système d'imagerie acousto-optique comprenant :

- un réseau de transducteurs ultrasonores régulièrement distribués spatialement,
- un dispositif d'émission lumineuse,
- un détecteur de lumière,
- un dispositif de commande configuré pour acquérir, par le détecteur de lumière, une pluralité de signaux de mesure $S_{mj}(t)$ associés à des ondes acoustiques non-focalisées modulées spatialement se propageant respectivement selon des directions de propagation m différentes,

le dispositif de commande étant configuré pour :

- faire émettre lesdites ondes acoustiques non focalisées modulées spatialement dans la zone d'observation successivement J fois avec respectivement J modulations spatiales périodiques j d'amplitude ayant une période spatiale identique selon au moins une direction de périodicité spatiale, et correspondant à un nombre donné P de transducteurs, les modulations spatiales périodiques j d'amplitude étant décalées spatialement en phase deux à deux,
- faire émettre par le dispositif d'émission lumineuse dans la zone d'observation, en même temps que chacune desdites ondes acoustiques non focalisées modulées spatialement, au moins une onde lumineuse incidente pour générer des onde lumineuses marquées modulées spatialement, chaque onde lumineuse marquée modulée spatialement comportant au moins une composante acousto-optique décalée en fréquence par au moins une desdites ondes acoustiques non-focalisées modulées spatialement,
- acquérir par le réseau de transducteurs, pour chaque onde lumineuse marquée modulée spatialement un signal de mesure $S_{mj}(t)$ correspondant à la modulation spatiale périodique j d'amplitude et à l'onde acoustique non-focalisée se propageant dans la direction m,
- procéder à une démodulation spatiale en combinant les J signaux de mesure $S_{mj}(t)$ correspondant aux différentes modulations spatiales périodiques d'amplitude pour une même direction de propagation m, pour obtenir un signal démodulé $S_m(t)$ propre à la direction de propagation m,
- déterminer une image d'au moins une partie de la zone d'observation à partir des signaux démodulés $S_i(t)$.

Le système d'imagerie acousto-optique peut en outre inclure l'une et / ou l'autre des caractéristiques suivantes :

- le réseau de transducteurs est linéaire et s'étend selon ladite direction de périodicité spatiale ;
- lesdites modulations spatiales périodiques d'amplitude sont des fonctions binaires, de sorte que seuls certains transducteurs ultrasonores sont activés pour une modulation spatiale périodique j d'amplitude ;
- J est égal à 4, la période spatiale correspond à un nombre P de transducteurs multiple de 4 et le dispositif de commande est configuré pour décaler spatialement en phase deux à deux lesdites modulations spatiales périodiques j d'amplitude d'une phase correspondant au quart de ladite période spatiale;

- le dispositif de commande est configuré pour calculer les transformées de Fourier temporelles $\check{S}_{mj}(v)$ des signaux de mesure $S_{mj}(t)$, $v$ étant la fréquence temporelle ; déterminer un signal $\check{S}_m(v)$ par une combinaison linéaire desdites transformées de Fourier temporelles $\check{S}_{mj}(v)$ ; et déterminer le signal démodulé $S_m(t)$ par transformée de Fourier inverse du signal $\check{S}_m(v)$.
- le dispositif de commande est configuré pour calculer le signal démodulé $S_m(t)$ par transformée de Fourier inverse d'un signal $\check{S}_m(v)$, avec :

$$\tilde{S}_m(v) = \tilde{S}_{0m0}(v) + \tilde{S}_{1m0}(v) + \tilde{S}_{-1m0}(v) \qquad (17),$$

où :

$$\tilde{S}_{0m0}(v) = 1/4\big[\tilde{S}_{m0}(v) + \tilde{S}_{m1}(v) + \tilde{S}_{m2}(v) + \tilde{S}_{m3}(v)\big] \qquad (14)$$

$$\tilde{S}_{1m0}(v) = 1/4\big[\tilde{S}_{m0}(v) - \tilde{S}_{m2}(v) - i(\tilde{S}_{m1}(v) - \tilde{S}_{m3}(v))\big] \qquad (15)$$

$$\tilde{S}_{-1m0}(v) = 1/4\big[\tilde{S}_{m0}(v) - \tilde{S}_{m2}(v) + i(\tilde{S}_{m1}(v) - \tilde{S}_{m3}(v))\big] \qquad (16)$$

les termes $\check{S}_{mj}(v)$ sont les transformées de Fourier temporelles des signaux de mesure $S_{mj}(t)$,
j est un entier compris entre 0 et 3,
v est la fréquence temporelle ;

- le dispositif de commande est configuré pour acquérir L signaux de mesure bruts $S_{ijl}(t)$ associés à chaque direction de propagation m d'une onde acoustique non-focalisée et à chaque modulation spatiale périodique j d'amplitude, et dans lequel lesdits L signaux de mesure $S_{mjl}(t)$ sont moyennés ensemble pour déterminer le signal de mesure $S_{mj}(t)$ utilisé pour la démodulation spatiale.

BREVE DESCRIPTION DES DESSINS

D'autres caractéristiques et avantages de l'invention apparaîtront au cours de la description suivante d'une de ses formes de réalisation, donnée à titre d'exemple non limitatif, en regard des dessins joints.
Sur les dessins :

- la figure 1 est une représentation schématique d'un exemple système d'imagerie acousto-optique tel que décrit précédemment,
- la figure 2 est un organigramme schématique d'un procédé d'imagerie acousto-optique mettant en oeuvre ce système,
- la figure 3 montre un exemple de motif formé par les transducteurs ultrasonores activés dans la mise en oeuvre du procédé de la figure 2, pour obtenir une modulation spatiale,
- la figure 4 illustre la modulation spatiale introduite par l'activation des transducteurs selon une série de motifs périodiques, et la démodulation ultérieure,
- les figures 5A et 5B illustrent des détails d'un mode de réalisation de l'étape de traitement du procédé de la figure 2,
- les figures 6a-6c montrent des images d'un même milieu obtenues par imagerie acousto-optique, respectivement avec des ondes acoustiques focalisées, avec des ondes planes sans modulation spatiale et avec des ondes planes modulées spatialement tel que décrit précédemment.

DESCRIPTION PLUS DETAILLEE

Sur les différentes figures, les mêmes références désignent des éléments identiques ou similaires.

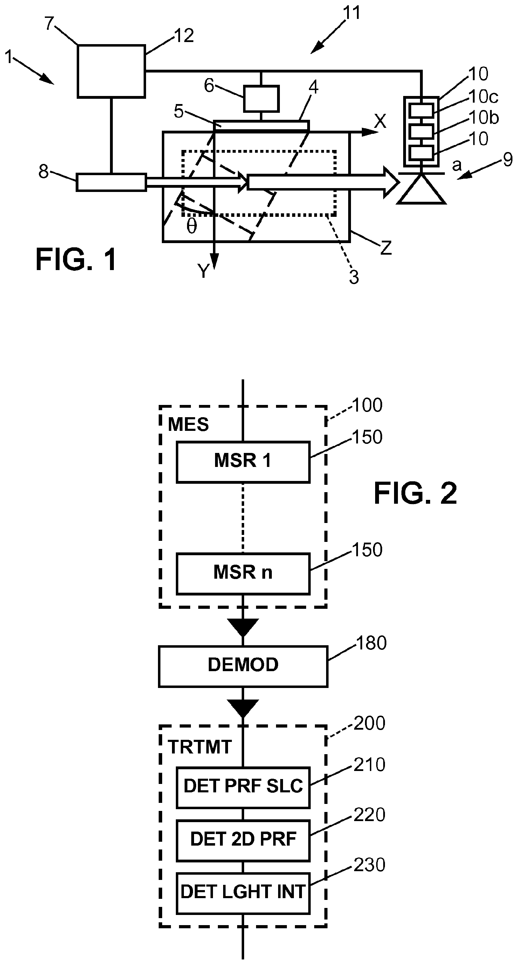

La figure 1 représente schématiquement un système d'imagerie acousto-optique 1 selon un mode de réalisation de l'invention.
On dispose ainsi d'un milieu 2, par exemple un objet ou un tissu biologique, à imager et comportant donc une zone d'observation 3. La zone d'observation 3 peut être en surface du milieu 2 mais peut éventuellement être localisée en profondeur dans le milieu 2, par exemple à quelques centimètres de profondeur.

Le milieu 2 est un milieu diffusant. Par « milieu diffusant », on entend notamment qu'au-delà d'une épaisseur caractéristique l* (libre parcours moyen de transport), qui est par exemple de l'ordre du millimètre dans les milieux biologiques, l'information que contient une onde lumineuse traversant le milieu est totalement brouillée et impossible à interpréter sans traitement. Ceci rend donc impossible une imagerie optique classique en profondeur. Ce phénomène est également appelé diffusion multiple de la lumière.

Un réseau 4 de transducteurs ultrasonores est en contact acoustique avec le milieu 2, soit directement en contact, soit par exemple couplé acoustiquement au milieu 2 par l'intermédiaire d'un élément de couplage comme une cuve remplie d'eau ou un coussin rempli d'eau.

Le réseau 4 de transducteurs ultrasonores est par exemple un réseau linéaire comportant par exemple quelques dizaines de transducteurs 5 (par exemple de 100 à 300). Les transducteurs 5 sont par exemple juxtaposés selon un axe X. Dans des variantes de réalisation, les transducteurs 5 pourront également être disposés en suivant une courbe, ou encore arrangés pour former une matrice bidimensionnelle. Dans un exemple particulier, le réseau 4 de transducteurs ultrasonores est un réseau linéaire de 192 transducteurs.

Le réseau 4 de transducteurs ultrasonores est commandé par des moyens de commande qui comportent par exemple une baie électronique 6 et un micro-ordinateur 7 contrôlant la baie électronique 6.

Le réseau 4 de transducteurs ultrasonores est ainsi apte à générer, dans la zone d'observation 3, une onde acoustique non-focalisée se propageant selon une direction de propagation prédéfinie. La direction de propagation peut être contrôlée de sorte à générer dans la zone d'observation 3 des ondes acoustiques non-focalisées se propageant selon des directions de propagation variées.

A titre non limitatif, le réseau 4 de transducteurs ultrasonores est par exemple apte à générer dans la zone d'observation 3 une onde ultrasonore ayant une fréquence centrale de l'ordre de quelques mégahertz, par exemple 6 MHz. Le réseau 4 de transducteurs ultrasonores est par exemple apte à générer dans la zone d'observation 3 une pluralité d'ondes ultrasonores ayant des directions de propagation choisies dans un secteur angulaire d'angle supérieur à 30 degrés, par exemple de 40 degrés.

Dans un mode de réalisation de l'invention, les ondes acoustiques non-focalisées sont des ondes acoustiques planes. Dans un autre mode de réalisation, les ondes acoustiques non-focalisées sont des ondes acoustiques divergentes, par exemple sphériques.

En pratique, les ondes acoustiques non-focalisées sont avantageusement des impulsions de largeur temporelle à mi-hauteur donnée, typiquement quelques $\mu$sec à quelques dizaines de $\mu$sec.

Le système 1 comporte également un dispositif d'émission lumineuse 8. Le dispositif d'émission lumineuse 8 est apte à émettre dans la zone d'observation 3 au moins une onde lumineuse incidente. En particulier, le dispositif d'émission lumineuse 8 est apte à l'émettre ladite onde lumineuse de manière simultanée avec l'émission d'une onde ultrasonore par le réseau 4 de transducteurs ultrasonores. Le dispositif d'émission lumineuse 8 est par exemple un laser ou de manière générale un dispositif d'émission permettant de contrôler le spectre de l'onde lumineuse incidente émise.

Par « onde lumineuse », on entend de manière large un rayonnement électromagnétique apte à se propager dans le milieu 2. On peut en particulier entendre un rayonnement électromagnétique appartenant au spectre infrarouge, visible ou ultraviolet.

Dans un exemple fourni à titre purement indicatif et non limitatif, le dispositif d'émission lumineuse 8 est un laser à semi-conducteur monofréquence amplifié de puissance 2 Watts et longueur d'onde 780 nanomètres (ce qui correspond donc à une fréquence incidente fi). La polarisation de l'onde lumineuse incidente peut également être contrôlée. Dans certains modes de réalisation, l'onde lumineuse peut être modulée temporellement et spatialement ou filtrée avant de pénétrer dans le milieu 2.

Le système 1 comporte encore un détecteur 9 apte à acquérir des signaux de mesure représentatifs des ondes lumineuses marquées. Le détecteur 9 est ainsi un photodétecteur sensible à une ou plusieurs longueurs d'onde électromagnétique correspondant à des longueurs d'onde de l'onde lumineuse marquée. Ainsi par exemple, le détecteur 9 est sensible à une composante acousto-optique générée par une interaction entre une onde lumineuse incidente et une onde acoustique non-focalisée se propageant dans la zone d'observation. Le détecteur 9 peut également être sensible à une composante porteuse, c'est-à-dire une composante de l'onde lumineuse marquée à la fréquence incidente fi.

Le détecteur 9 est par exemple une photodiode.

Le système 1 peut comporter des éléments de prétraitement ou de post traitement du signal 10, éventuellement intégrés dans le détecteur 9. Les éléments de post traitement du signal 10 peuvent par exemple comprendre un filtre passe-haut 10a, un amplificateur large bande 10b (par exemple Thorlabs, DHPVA) et un convertisseur analogique numérique 10c.

Ainsi en particulier, le signal de mesure peut être échantillonné par le convertisseur analogique numérique 10c à une fréquence supérieure à quelques mégahertz, de préférence supérieur à dix mégahertz, par exemple une fréquence d'échantillonnage de 40 MHz.

De cette manière, chaque signal de mesure peut notamment comporter une série temporelle de valeurs d'intensité lumineuse d'une composante acousto-optique d'une onde lumineuse marquée décalée en fréquence par une onde acoustique non-focalisée.

Le réseau de transducteurs 4, le dispositif d'émission lumineuse 8 et le détecteur 9 peuvent ainsi former un dispositif d'acquisition 11 d'un système 1 selon l'invention. Un tel dispositif d'acquisition 11 est notamment apte à acquérir une pluralité de signaux de mesure associée à une pluralité d'ondes acoustiques non-focalisées comme cela va être détaillé ci-après.

Un procédé d'imagerie acousto-optique d'une zone d'observation d'un milieu est notamment illustré plus en détail sur la figure 2 et peut par exemple être mis en oeuvre, au moyen du système 1, de la manière suivante.

**(a) Etape de mesure**

Au cours d'une étape de mesure 100 (MES), on peut acquérir une pluralité de signaux de mesure associée à une pluralité d'ondes acoustiques (ultrasonores) non-focalisées.

L'étape d'acquisition 100 comporte une pluralité d'opérations de mesure 150.

Au cours de chaque opération de mesure 150, on procède aux sous étapes suivantes :

**(a1) Emission acoustique**

On génère, dans la zone d'observation 3, au moyen du réseau de transducteurs 4, des ondes acoustiques non-focalisées, par exemple des impulsions, se propageant selon une direction de propagation faisant un angle $\theta$ avec l'axe Y perpendiculaire à la direction X dans le plan de l'image (qui est également l'angle entre l'axe X et les droites équiphases dans le cas des ondes planes). Les ondes acoustiques sont émises selon plusieurs directions de propagations, désignées chacune par un indice m, faisant chacune un angle $\theta_m$ avec l'axe Y. Les directions de propagation m peuvent couvrir un secteur angulaire compris entre 30 et 50 degrés, par exemple 40 degrés. Les directions de propagation m des ondes acoustiques non-focalisées peuvent être séparées par un pas angulaire compris entre 0.5 degré et 2 degrés, par exemple 1 degré. Dans le cas d'une étendue angulaire de 40 degrés et d'un pas de 1 degré, le nombre M de directions de propagations est donc de M = 41.

De plus, pour une même direction de propagation m, on émet l'onde acoustique J fois en appliquant à chaque fois une modulation spatiale j d'amplitude aux transducteurs 5 du réseau 4. Les modulations spatiales j d'amplitude sont périodiques, avec une période spatiale de P transducteurs $T_k$ dans la direction X (les transducteurs $T_k$ étant répartis régulièrement, cette période spatiale exprimée en nombre de transducteurs est équivalente à une certaine distance xp). Les modulations spatiales d'amplitude j sont décalées spatialement selon la direction X l'une par rapport à l'autre.

La modulation spatiale d'amplitude susmentionnée correspond à une fonction spatiale A(k), périodique dans + direction X, telle que le signal $e_{mjk}(t)$ émis par chaque transducteur $T_k$ ait une amplitude égale à $A(k).A_0$, où $A_0$ est un nombre prédéfini.

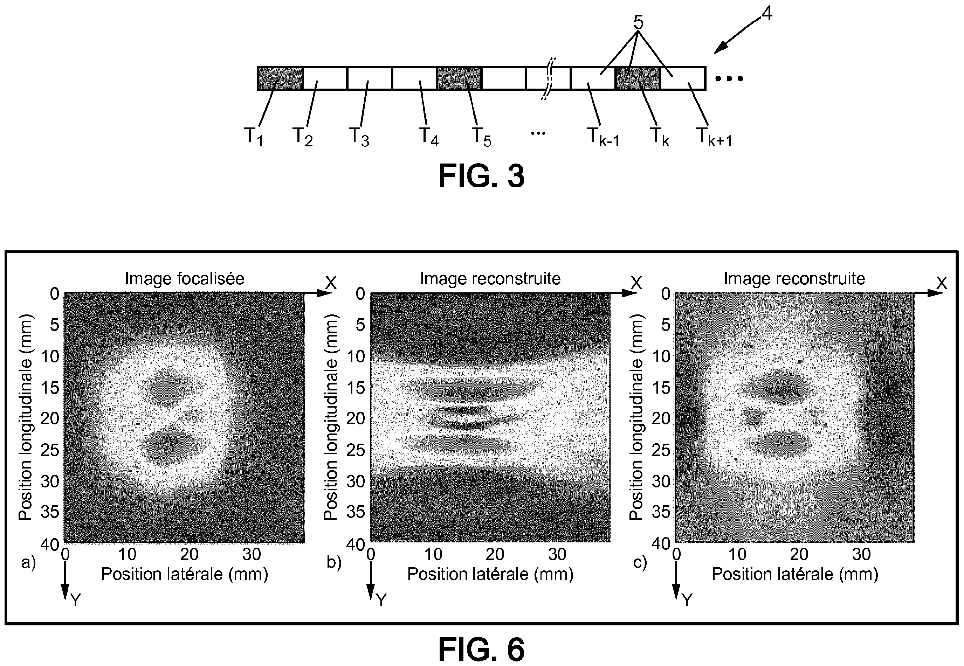

Selon une forme de réalisation illustrée à la figure 3, la modulation spatiale d'amplitude A(k) est une fonction binaire valant soit 0 soit 1 suivant les transducteurs $T_k$, de sorte que seuls certains transducteurs ultrasonores sont activés pour une modulation spatiale périodique j donnée.

Comme illustré sur la figure 3, les transducteurs 5 ainsi activés à chaque émission sont disposés selon un motif périodique, ayant ladite période spatiale de P transducteurs $T_k$ dans la direction X.

Le motif formé par les transducteurs $T_k$ activés pour chaque modulation spatiale périodique j d'amplitude, peut être divers. Dans l'exemple de la figure 3, on active simplement un transducteur $T_k$ sur 4 (pour un réseau de 192 transducteurs, on active donc 48 transducteurs pour chaque modulation spatiale périodique j d'amplitude), soit :

- pour j=0 : on active les transducteurs T1, T5, etc (colorés en noir sur la figure 3).
- pour j=1 : on active les transducteurs T2, T6, etc.
- pour j=2 : on active les transducteurs T3, T7, etc.
- pour j=3 : on active les transducteurs T4, T8, etc.

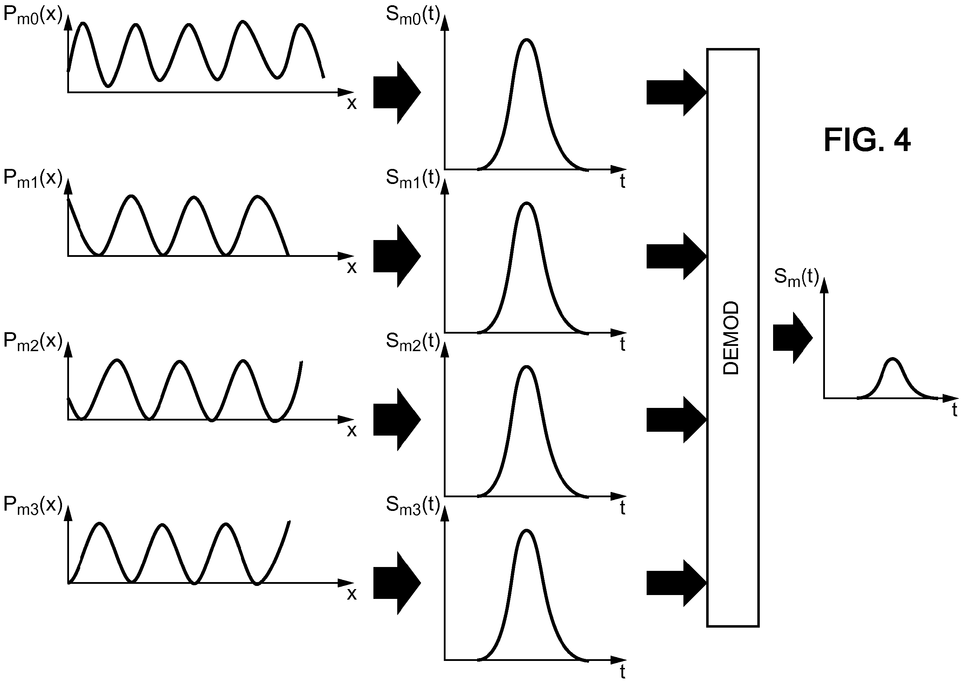

Comme illustré sur la figure 4, les modulations spatiales périodiques j d'amplitude induisent une variation périodique selon X de la pression $P_{mj}(t)$ générées par les ondes ultrasonores, et cette courbe de pression est décalée latéralement de P/4 selon X d'une modulation spatiale périodique j d'amplitude à l'autre.

**(a2) Emission lumineuse**

Pendant l'émission acoustique, on émet, dans la zone d'observation 3, au moyen du moyen dispositif d'émission lumineuse 8, une onde lumineuse incidente pour générer une onde lumineuse marquée comportant au moins une composante acousto-optique décalée en fréquence par l'onde acoustique non-focalisée.

**(a3) Acquisition**

Pour chaque direction de propagation m et modulation spatiale périodique j d'amplitude, on acquière au moyen du détecteur 9, un signal de mesure $S_{mj}(t)$ (t désignant le temps) représentatif de l'onde lumineuse marquée.

Au total, on acquière n = M*J signaux $S_{mj}(t)$ dans l'étape de mesure 100. Pour M = 41 et J = 4, on acquière donc n = 164 signaux $S_{mj}(t)$.

Dans un mode de réalisation de l'invention, chaque opération de mesure 150 est répétée L fois pour acquérir L signaux de mesure $S_{mjl}(t)$ qui sont ensuite moyennés ensemble pour chaque valeur de m et j pour obtenir des signaux $S_{mj}(t)$ utilisés dans la suite du traitement. L peut par exemple être supérieur à dix, par exemple une centaine de fois ou un millier de fois. Pour M = 41, J = 4 et L = 1000, on procède donc à 164 000 tirs d'ondes acoustiques pour obtenir une image du milieu 2.

**(b)Démodulation spatiale**

Comme représenté sur les figures 2 et 4, les signaux $S_{mj}(t)$ sont ensuite démodulés spatialement dans une étape de démodulation 180 (DEMOD) . Plus précisément, les signaux $S_{mj}(t)$ correspondant aux différentes modulations spatiales périodiques j d'amplitude pour une même direction de propagation m, sont combinés pour obtenir un signal $S_m(t)$.

Dans l'exemple susmentionné où J=4 et le décalage spatial entre motifs est de P/4, $S_m(t)$ peut être obtenu par démodulation 4 phases comme expliqué ci-après.

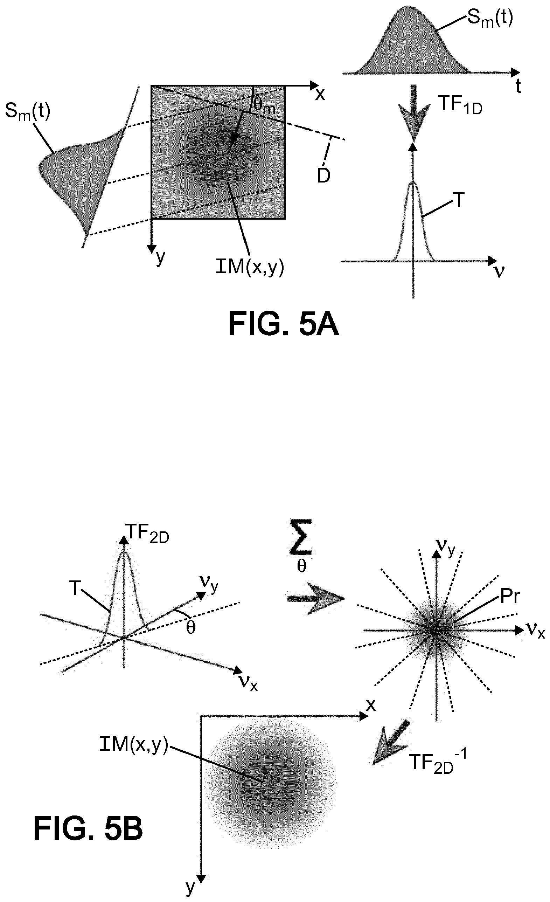

Chaque onde ultrasonore m se propage dans le milieu 2. Dans le cas des ondes planes, les équiphases de cette onde m sont des droites D, inclinées d'un angle $\theta_m$ par rapport à la direction X (voir Figure 5A) qui correspondent au lieu des points atteints par l'onde au bout d'un temps t, correspondant à une distance $V_{US}.t$, où $V_{US}$ est la vitesse de l'onde ultrasonore. On joue sur le retard d'excitation des transducteurs $T_k$ (selon X) pour créer l'inclinaison de l'onde incidente. Pour ce faire, chaque transducteur $T_k$ d'abscisse x a par exemple un retard $xsin\theta_m/V_{US}$ par rapport à un élément de référence.

Le lieu des points atteints par l'onde plane au bout d'un temps t est donc donné par l'équation :

$$xsin\theta_m \ + \ ycos\theta_m \ = \ V_{US}.t \quad (1)$$

A titre d'exemple, la pression générée par les ondes ultrasonores émises peut être modulée selon X, de façon proportionnelle à :

$$1/2[1 \ + \ \cos(2\pi\eta x \ + \ \varphi_j)],$$

où:

- $\eta$ = 1/xp est la fréquence spatiale de la modulation spatiale d'amplitude ;
- $\varphi_j$ est la phase spatiale de la modulation spatiale d'amplitude j (dans le cas où J = 4, $\varphi_j$ est égal à j.$\pi$/2, j étant un entier compris entre 0 et 3).

Par projection, la fréquence spatiale effective le long de la direction de l'onde m sera donnée par :

$$\eta_m \ = \ \eta \ / \ cos\theta_m \quad (2)$$

On aura donc le long des équiphases (droites D) une modulation effective de la pression générée par l'onde de la forme :

$$[1 + \cos(2\pi\eta_m(xcos\theta_m - ysin\theta_m) + \varphi_j] \quad (3)$$

On peut donc écrire la pression spatio-temporelle de la manière suivante :

$$P_{US}(\vec{r}, t) = \frac{1}{2} f\left(t - \frac{xsin\theta_m \ + \ ycos\theta_m}{V_{US}}\right) . \left[1 \ + \ \cos\left(2\pi\eta_m(xcos\theta_m \ - \ ysin\theta_m) \ + \ \varphi_j\right)\right] . \cos(2\pi\nu_{US}.t) \quad (4)$$

où :

- $v_{US}$ est la fréquence temporelle des ultrasons,
- f(t) représente la forme temporelle de l'excitation ultrasonore appliquée à chaque transducteur 5.

Le signal capté $S_{mj}(t)$ des photons marqués s'écrit donc :

$$S_{mj}(t) \propto \frac{1}{4} \iint IN(x,y) f^2\left(t - \frac{x\sin\theta_m + y\cos\theta_m}{v_{US}}\right) \cdot \left[1 + \cos(2\pi\eta_m(x\cos\theta_m - y\sin\theta_m) + \varphi_j)\right]^2 dxdy$$

(5)

où :

- IN(x,y) est le signal de l'image à reconstituer (intensité locale du signal optique dans le plan XY).

La transformée de Fourier temporelle de ce signal donne alors (coupe centrale) :

$$\tilde{S}_{mj}(v) \propto \frac{F(v)}{4} \iint IN(x,y) \cdot \left[1 + \cos(2\pi\eta_m(x\cos\theta_m - y\sin\theta_m) + \varphi_j)\right]^2 e^{-i2\pi v\frac{x\sin\theta_m + y\cos\theta_m}{v_{US}}} dxdy$$

(6)

Où :

- v désigne la fréquence temporelle,
- F(v) désigne la transformée de fourrier temporelle de la fonction $f^2(t)$.

En développant la structure spatiale, on obtient :

$$\tilde{S}_{mj}(v) \propto \frac{F(v)}{4} \iint IN(x,y) \left(\frac{3}{2} + 2\cos(2\pi\eta_m(x\cos\theta_m - y\sin\theta_m) + \varphi_j) + \right.$$

$$\left. \frac{1}{2}\cos(2\pi.2\eta_m(x\cos\theta_m - y\sin\theta_m) + 2\varphi_j) \right) e^{-i2\pi v\frac{x\sin\theta_m + y\cos\theta_m}{v_{US}}} dxdy \quad (7)$$

Cette intégrale est la somme de 5 termes :

$$\tilde{S}_{mj}(v) = \tilde{S}_{0mj}(v) + \tilde{S}_{1mj}(v) + \tilde{S}_{-1mj}(v) + \tilde{S}_{2mj}(v) + \tilde{S}_{-2mj}(v) \quad (8)$$

avec :

$$\tilde{S}_{0mj}(v) = \frac{3F(v)}{8} \iint IN(x,y) e^{-i2\pi v\frac{x\sin\theta_m + y\cos\theta_m}{v_{US}}} dxdy \quad (9)$$

$$\tilde{S}_{1mj}(v) = e^{i\varphi_j} \frac{F(v)}{4} \iint IN(x,y) e^{-i2\pi x\left(\frac{v\sin\theta_m}{v_{US}} + \eta_m\cos\theta_m\right)} e^{-i2\pi y\left(\frac{v\cos\theta_m}{v_{US}} - \eta_m\sin\theta_m\right)} dxdy$$

(10)

$$\tilde{S}_{-1mj}(v) = e^{-i\varphi_j} \frac{F(v)}{4} \iint IN(x,y) e^{-i2\pi x\left(\frac{v\sin\theta_m}{v_{US}} - \eta_m\cos\theta_m\right)} e^{-i2\pi y\left(\frac{v\cos\theta_m}{v_{US}} + \eta_m\sin\theta_m\right)} dxdy$$

(11)

$$\tilde{S}_{2mj}(v) = e^{2i\varphi_j}\ \frac{F(v)}{16}\iint IN(x,y)\,e^{-i2\pi x\left(\frac{\upsilon\sin\theta_m}{V_{US}}+2\eta_m\cos\theta_m\right)}\,e^{-i2\pi y\left(\frac{\upsilon\cos\theta_m}{V_{US}}-2\eta_m\sin\theta_m\right)}\,dxdy$$
$$(12)$$

$$\tilde{S}_{-2mj}(v) =$$
$$e^{-2i\varphi_j}\ \frac{F(v)}{16}\iint IN(x,y)\,e^{-i2\pi x\left(\frac{\upsilon\sin\theta_m}{V_{US}}-2\eta_m\cos\theta_m\right)}\,e^{-i2\pi y\left(\frac{\upsilon\cos\theta_m}{V_{US}}+2\eta_m\sin\theta_m\right)}\,dxdy \quad (13)$$

Chaque terme $\tilde{S}_{pmj}(v)$ correspond à la projection du signal sur un vecteur fréquence spatiale.

Pour chaque direction de propagation m, on enregistre le signal pour par exemple 4 valeurs de la phase $\varphi_j$, qui correspondent à des décalages spatiaux de la structure de pression dans le milieu 2.

On peut extraire les termes pertinents (p=0,1,-1) comme suit :

$$\tilde{S}_{0m0}(v) = 1/4\left[\tilde{S}_{m0}(v) + \tilde{S}_{m1}(v) + \tilde{S}_{m2}(v) + \tilde{S}_{m3}(v)\right] \qquad (14)$$

$$\tilde{S}_{1m0}(v) = 1/4\left[\tilde{S}_{m0}(v) - \tilde{S}_{m2}(v) - i(\tilde{S}_{m1}(v) - \tilde{S}_{m3}(v))\right] \qquad (15)$$

$$\tilde{S}_{-1m0}(v) = 1/4\left[\tilde{S}_{m0}(v) - \tilde{S}_{m2}(v) + i(\tilde{S}_{m1}(v) - \tilde{S}_{m3}(v))\right] \qquad (16)\,.$$

En pratique, le terme $\tilde{S}_{0m0}(v)$ correspondant à p = 0 comprend les informations obtenues avec des ondes planes non modulées spatialement, les termes correspondant à p=1 et -1 (harmoniques simples) apportent des informations supplémentaires dans l'espace des fréquences spatiales et permettent d'améliorer la qualité de la reconstruction du signal. Les termes correspondant à p=2 et -2 (harmoniques doubles) sont de préférence éliminés pour optimiser la qualité du signal de reconstruction.

On somme les trois signaux ci-dessus pour obtenir un signal $\check{S}_m(v)$ pour chaque inclinaison m :

$$\tilde{S}_m(v) = \tilde{S}_{0m0}(v) + \tilde{S}_{1m0}(v) + \tilde{S}_{-1m0}(v) \qquad (17)\,,$$

puis on en déduit un signal démodulé $S_m(t)$ par transformée de Fourier inverse de $\check{S}_m(v)$.

**(c) Traitement**

Le procédé comporte par la suite une étape de traitement 200 au cours de laquelle on détermine une image de la zone d'observation 3 dont les pixels sont représentatifs d'une intensité lumineuse dans la zone d'observation, à partir des signaux démodulés $S_m(t)$.

Cette étape de traitement peut être effectuée comme décrit dans le document WO2016193554 susmentionné.

A titre non limitatif, Cette étape de traitement 200 comprend avantageusement la mise en oeuvre d'une transformation de Radon comme illustré sur les figures 5A et 5B. L'étape de traitement peut également comporter une double transformée de Fourier (temporelle, puis spatiale).

De manière schématique, l'étape de traitement 200 (TRTMT) peut comprendre les opérations suivantes :

- déterminer (210 - DET PRF SLC) une pluralité de tranches de profil associée à la pluralité de signaux démodulés $S_i(t)$ (figure 5A),
- déterminer (220 - DET 2D PRF) un profil bidimensionnel à partir de la pluralité de tranches de profil (figure 5B), et
- déterminer (230 - DET LGHT INT) au moins une valeur représentative d'une intensité lumineuse dans la zone d'observation à partir du profil bidimensionnel (également figure 5B).

Plus précisément, on commence par déterminer, pour chaque signal démodulés $S_i(t)$, une tranche de profil T.

Pour cela, on met en oeuvre une transformation de Fourier unidimensionnelle du signal démodulés $S_i(t)$ qui fournit la tranche de profil T associée comme illustré sur la figure 5A.

Puis, à partir de la pluralité de tranches de profil T associées à la pluralité de signaux démodulés $S_i(t)$, on détermine un profil bidimensionnel P. Comme cela est illustré sur la figure 5B, le profil bidimensionnel P est déterminé par recalage dans un espace de Fourier de la pluralité de tranches de profil. Chaque tranche de profil T est ainsi recalée dans l'espace de Fourier en fonction de la direction de propagation m de l'onde acoustique non-focalisée qui était associée au signal démodulés $S_m(t)$ associé à la tranche de profil T.

Ainsi, il est par exemple possible de recaler les tranches de profil T pour emplir le secteur angulaire constitué par les directions de propagation des ondes non-focalisées.

Une fois le profil bidimensionnel P obtenu, il est alors possible de déterminer une ou plusieurs valeur(s) représentative(s) d'une intensité lumineuse IN(x,y) dans la zone d'observation 3 par une transformation de Fourier bidimensionnelle inverse du profil bidimensionnel Pr, comme illustré également sur la figure 5B.

Dans des modes de réalisation de l'invention, les tranches de profil T peuvent être complétées pour déterminer le profil bidimensionnel Pr.

La figure 6c illustre une image d'un milieu comportant deux inclusions obtenu lors de la mise en oeuvre d'un exemple de réalisation du procédé décrit ci-dessus (avec M = 41, J = 4, L = 1000). Les figures 6a et 6b sont des images du même milieu obtenues respectivement par imagerie acousto-optique avec des ondes acoustiques focalisées et par imagerie acousto-optique telle que décrite dans le document WO2016193554 susmentionné.

Par rapport à l'image de la figure 6b, l'amélioration de résolution latérale est nette. Par rapport à l'image de la figure 6a, la résolution est comparable mais l'image de la figure 5c peut être obtenue environ 10 fois plus rapidement que celle de la figure 6a.

**Revendications**

1. Procédé d'imagerie acousto-optique pour imager une zone d'observation (3) d'un milieu (2), le procédé comprenant :

   - une étape de mesure (100) au cours de laquelle on acquière par voie optique une pluralité de signaux de mesure $S_{mj}(t)$ associés à des ondes acoustiques non-focalisées se propageant respectivement selon des directions de propagation m différentes, lesdites ondes acoustiques non focalisées étant émises dans la zone d'observation (3) respectivement par un réseau (4) de transducteurs ultrasonores ($T_k$) régulièrement distribués spatialement, lesdites ondes acoustiques non focalisées étant émises, dans chaque direction de propagation m, successivement J fois avec respectivement J modulations spatiales périodiques j d'amplitude pour former des ondes acoustiques non focalisées modulées spatialement, les modulations spatiales périodiques j d'amplitude ayant une période spatiale identique selon au moins une direction (X) de périodicité spatiale, et correspondant à un nombre donné P de transducteurs, les modulations spatiales périodiques j d'amplitude étant décalées spatialement en phase deux à deux, l'étape de mesure comportant, pour chaque modulation spatiale périodique j d'amplitude, une pluralité d'opérations de mesure (150) successives comprenant les sous-étapes suivantes :

      o une sous-étape d'émission acoustique dans laquelle on fait émettre une onde acoustique non-focalisée modulée spatialement avec ladite modulation spatiale périodique j d'amplitude et se propageant selon une direction de propagation m,
      o une sous-étape d'émission lumineuse dans laquelle on émet dans la zone d'observation (3), en même temps que l'onde acoustique non-focalisée modulée spatialement, une onde lumineuse incidente pour générer une onde lumineuse marquée modulée spatialement, comportant au moins une composante acousto-optique décalée en fréquence respectivement par l'onde acoustique non-focalisée modulée spatialement,
      o une sous-étape d'acquisition dans laquelle on capte l'onde lumineuse marquée modulée spatialement et on acquiert ainsi un signal de mesure $S_{mj}(t)$ correspondant à la modulation spatiale périodique j d'amplitude et à l'onde acoustique non-focalisée se propageant dans la direction m,

   - une étape de démodulation spatiale, au cours de laquelle on combine les différents signaux de mesure $S_{mj}(t)$ correspondant aux J modulations spatiales périodiques d'amplitude pour une même direction de propagation m, pour obtenir un signal démodulé $S_m(t)$ propre à la direction de propagation m,
   - une étape de traitement (200) au cours de laquelle on détermine une image d'au moins une partie de la zone d'observation (3) à partir des signaux démodulés $S_m(t)$.

2. Procédé d'imagerie acousto-optique selon la revendication 1, dans lequel le réseau (4) de transducteurs est linéaire et s'étend selon ladite direction de périodicité spatiale (X).

3. Procédé d'imagerie acousto-optique selon la revendication 1 ou la revendication 2, dans lequel lesdites modulations spatiales périodiques j d'amplitude sont des fonctions binaires, de sorte que seuls certains transducteurs ultrasonores ($T_k$) sont activés pour une modulation spatiale périodique j d'amplitude donnée.

4. Procédé d'imagerie acousto-optique selon l'une quelconque des revendications précédentes, dans lequel J est égal à 4, ladite période spatiale correspond à un nombre P de transducteurs multiple de 4, et les modulations spatiales périodiques j d'amplitude sont décalées spatialement en phase deux à deux d'une phase correspondant au quart de ladite période spatiale.

5. Procédé d'imagerie acousto-optique selon la revendication 4, dans lequel, au cours de l'étape de démodulation spatiale, on calcule le signal démodulé $S_m(t)$ par transformée de Fourier inverse d'un signal $\tilde{S}_m(\nu)$, avec :

$$\tilde{S}_m(\nu) = \tilde{S}_{0m0}(\nu) + \tilde{S}_{1m0}(\nu) + \tilde{S}_{-1m0}(\nu) \qquad (17),$$

où :

$$\tilde{S}_{0m0}(\nu) = 1/4\big[\tilde{S}_{m0}(\nu) + \tilde{S}_{m1}(\nu) + \tilde{S}_{m2}(\nu) + \tilde{S}_{m3}(\nu)\big] \qquad (14)$$

$$\tilde{S}_{1m0}(\nu) = 1/4\big[\tilde{S}_{m0}(\nu) - \tilde{S}_{m2}(\nu) - i(\tilde{S}_{m1}(\nu) - \tilde{S}_{m3}(\nu))\big] \qquad (15)$$

$$\tilde{S}_{-1m0}(\nu) = 1/4\big[\tilde{S}_{m0}(\nu) - \tilde{S}_{m2}(\nu) + i(\tilde{S}_{m1}(\nu) - \tilde{S}_{m3}(\nu))\big] \qquad (16)$$

les termes $\tilde{S}_{mj}(\nu)$ sont les transformées de Fourier temporelles des signaux de mesure $S_{mj}(t)$,
j est un entier compris entre 0 et 3,
ν est la fréquence temporelle.

6. Procédé d'imagerie acousto-optique selon l'une quelconque des revendications précédentes, dans lequel l'onde acoustique non-focalisée est choisie parmi une onde acoustique plane et une onde acoustique divergente.

7. Procédé d'imagerie acousto-optique selon l'une quelconque des revendications précédentes, dans lequel les directions de propagation m des ondes acoustiques non-focalisées couvrent un secteur angulaire d'angle compris entre 30 et 50 degrés.

8. Procédé d'imagerie acousto-optique selon l'une quelconque des revendications précédentes, dans lequel les directions de propagation m des ondes acoustiques non-focalisées sont séparées par un pas angulaire compris entre 0.5 degré et 2 degrés.

9. Procédé d'imagerie acousto-optique selon l'une quelconque des revendications précédentes, dans lequel chaque opération de mesure (150) est réitérée L fois pour acquérir L signaux de mesure bruts $S_{ijl}(t)$ associés à chaque direction de propagation m d'une onde acoustique non-focalisée et à chaque modulation spatiale périodique j d'amplitude, et dans lequel lesdits L signaux de mesure bruts $S_{mjl}(t)$ sont moyennés ensemble pour déterminer le signal de mesure $S_{mj}(t)$ utilisé pour l'étape de démodulation spatiale (180).

10. Système (1) d'imagerie acousto-optique pour imager une zone d'observation (3) d'un milieu (2), le système d'imagerie acousto-optique comprenant :

   - un réseau (4) de transducteurs ultrasonores ($T_k$) régulièrement distribués spatialement,
   - un dispositif d'émission lumineuse (8),
   - un détecteur de lumière (9),
   - un dispositif de commande configuré pour acquérir, par le détecteur de lumière (9), une pluralité de signaux de mesure $S_{mj}(t)$ associés à des ondes acoustiques non-focalisées modulées spatialement se propageant respectivement selon des directions de propagation m différentes,

   le dispositif de commande étant configuré pour :

- faire émettre lesdites ondes acoustiques non focalisées modulées spatialement dans la zone d'observation (3) successivement J fois, avec respectivement J modulations spatiales périodiques j d'amplitude ayant une période spatiale selon au moins une direction de périodicité spatiale (X) identique, et correspondant à un nombre donné P de transducteurs, les modulations spatiales périodiques j d'amplitude étant décalées spatialement en phase deux à deux,

- faire émettre par le dispositif d'émission lumineuse (8) dans la zone d'observation (3), en même temps que chacune desdites ondes acoustiques non focalisées modulées spatialement, au moins une onde lumineuse incidente pour générer des ondes lumineuses marquées modulées spatialement, chaque onde lumineuse marquée modulée spatialement comportant au moins une composante acousto-optique décalée en fréquence par au moins une desdites ondes acoustiques non-focalisées modulées spatialement,

- acquérir par le réseau (4) de transducteurs, pour chaque onde lumineuse marquée modulée spatialement un signal de mesure $S_{mj}(t)$ correspondant à la modulation spatiale périodique j d'amplitude et à l'onde acoustique non-focalisée se propageant dans la direction m,

- procéder à une démodulation spatiale en combinant les J signaux de mesure $S_{mj}(t)$ correspondant aux différentes modulations spatiales périodiques d'amplitude pour une même direction de propagation m, pour obtenir un signal démodulé $S_m(t)$ propre à la direction de propagation m,

- déterminer une image d'au moins une partie de la zone d'observation (3) à partir des signaux démodulés $S_m(t)$.

**11.** Système d'imagerie acousto-optique selon la revendication 10, dans lequel le réseau (4) de transducteurs est linéaire et s'étend selon ladite direction de périodicité spatiale (X).

**12.** Système d'imagerie acousto-optique selon la revendication 10 ou la revendication 11, dans lequel lesdites modulations spatiales périodiques d'amplitude sont des fonctions binaires, de sorte que seuls certains transducteurs ultrasonores ($T_k$) sont activés pour une modulation spatiale périodique j d'amplitude donnée.

**13.** Système d'imagerie acousto-optique selon l'une quelconque des revendications 10 à 12, dans lequel J est égal à 4, ladite période spatiale correspond à un nombre P de transducteurs multiple de 4 et le dispositif de commande est configuré pour décaler spatialement en phase deux à deux lesdites modulations spatiales périodiques j d'amplitude d'une phase correspondant au quart de ladite période spatiale.

**14.** Système d'imagerie acousto-optique selon la revendication 13, dans lequel le dispositif de commande est configuré pour calculer le signal démodulé $S_m(t)$ par transformée de Fourier inverse d'un signal $\check{S}_m(\nu)$, avec :

$$\tilde{S}_m(\nu) = \tilde{S}_{0m0}(\nu) + \tilde{S}_{1m0}(\nu) + \tilde{S}_{-1m0}(\nu) \qquad (17),$$

où :

$$\tilde{S}_{0m0}(\nu) = 1/4[\tilde{S}_{m0}(\nu) + \tilde{S}_{m1}(\nu) + \tilde{S}_{m2}(\nu) + \tilde{S}_{m3}(\nu)] \qquad (14)$$

$$\tilde{S}_{1m0}(\nu) = 1/4[\tilde{S}_{m0}(\nu) - \tilde{S}_{m2}(\nu) - i(\tilde{S}_{m1}(\nu) - \tilde{S}_{m3}(\nu))] \qquad (15)$$

$$\tilde{S}_{-1m0}(\nu) = 1/4[\tilde{S}_{m0}(\nu) - \tilde{S}_{m2}(\nu) + i(\tilde{S}_{m1}(\nu) - \tilde{S}_{m3}(\nu))] \qquad (16)$$

les termes $\check{S}_{mj}(\nu)$ sont les transformées de Fourier temporelles des signaux de mesure $S_{mj}(t)$,
j est un entier compris entre 0 et 3,
$\nu$ est la fréquence temporelle.

**15.** Système d'imagerie acousto-optique selon l'une quelconque des revendications 10 à 14, dans lequel le dispositif de commande est configuré pour acquérir L signaux de mesure bruts $S_{mjl}(t)$ associés à chaque direction de propagation m d'une onde acoustique non-focalisée et à chaque modulation spatiale périodique j d'amplitude, et dans lequel lesdits L signaux de mesure $S_{mjl}(t)$ sont moyennés ensemble pour déterminer le signal de mesure $S_{mj}(t)$ utilisé pour la démodulation spatiale.

**Patentansprüche**

1. Akustooptisches Abbildungsverfahren zum Abbilden eines Beobachtungsbereichs (3) eines Mediums (2), wobei das Verfahren aufweist:

    einen Messschritt (100), in dessen Verlauf auf optischem Wege eine Vielzahl von Messsignalen $S_{mj}(t)$ erfasst wird, die nicht fokussierten akustischen Wellen zugeordnet sind, die sich jeweils in verschiedenen Ausbreitungsrichtungen m ausbreiten, wobei die nicht fokussierten akustischen Wellen in den Beobachtungsbereich (3) jeweils von einem Netz (4) von räumlich gleichmäßig verteilten Ultraschallwandlern ($T_k$) ausgesendet werden, wobei die nicht fokussierten akustischen Wellen in jeder Ausbreitungsrichtung m ausgesendet werden, nacheinander J-mal mit jeweils J periodischen räumlichen Amplitudenmodulationen j, um räumlich modulierte nicht fokussierte akustische Wellen zu bilden, wobei die periodischen räumlichen Amplitudenmodulationen j eine identische räumliche Periode in mindestens einer Richtung (X) der räumlichen Periodizität haben, und einer gegebenen Anzahl P von Wandlern entspricht, wobei die periodischen räumlichen Amplitudenmodulationen j paarweise räumlich phasenverschoben sind, wobei der Messschritt für jede periodische räumliche Amplitudenmodulation j eine Vielzahl von aufeinanderfolgenden Messoperationen (150) aufweist, die folgenden Unterschritte aufweisend:

    einen Unterschritt der akustischen Emission, in dem man eine nicht fokussierte akustische Welle, die mit der genannten periodischen räumlichen Amplitudenmodulation j räumlich moduliert ist und sich entlang einer Ausbreitungsrichtung m ausbreitet, zum Aussenden veranlasst,
    einen Unterschritt der Lichtemission, in dem man in den Beobachtungsbereich (3) gleichzeitig mit der räumlich modulierten, nicht fokussierten akustischen Welle eine einfallende Lichtwelle aussendet, um eine markierte, räumlich modulierte Lichtwelle zu erzeugen, die mindestens eine akustooptische Komponente aufweist, die jeweils durch die räumlich modulierte, nicht fokussierte akustische Welle frequenzverschoben ist,
    einen Unterschritt der Erfassung, in dem man die markierte räumlich modulierte Lichtwelle erfasst und so ein Messsignal $S_{mj}(t)$ erfasst, das der periodischen räumlichen Amplitudenmodulation j und der sich in Richtung m ausbreitenden nicht fokussierten akustischen Welle entspricht,

    einen Schritt der räumlichen Demodulation, bei dem die verschiedenen Messsignale $S_{mj}(t)$, die den J periodischen räumlichen Amplitudenmodulationen für eine gleiche Ausbreitungsrichtung m entsprechen, kombiniert werden, um ein demoduliertes Signal $S_m(t)$ zu erhalten, das der Ausbreitungsrichtung m eigen ist,
    einen Verarbeitungsschritt (200), in dessen Verlauf aus den demodulierten Signalen $S_m(t)$ ein Bild von mindestens einem Teil des Beobachtungsbereichs (3) bestimmt wird.

2. Akustooptisches Abbildungsverfahren nach Anspruch 1, bei dem das Netz (4) von Wandlern linear ist und sich in der genannten räumlichen Periodizitätsrichtung (X) erstreckt.

3. Akustooptisches Abbildungsverfahren nach Anspruch 1 oder 2, wobei die periodischen räumlichen Amplitudenmodulationen j binäre Funktionen sind, so dass nur bestimmte Ultraschallwandler ($T_k$) für eine gegebene räumlich-periodische Amplitudenmodulation j aktiviert werden.

4. Akustooptisches Abbildungsverfahren nach einem der vorhergehenden Ansprüche, wobei J gleich 4 ist, die räumliche Periode einer Anzahl P von Wandlern entspricht, die ein Vielfaches von 4 ist, und die periodischen räumlichen Modulationen j der Amplitude paarweise um eine Phase räumlich phasenverschoben sind, die einem Viertel der räumlichen Periode entspricht.

5. Akustooptisches Abbildungsverfahren nach Anspruch 4, wobei während des Schritts der räumlichen Demodulation das demodulierte Signal $S_m(t)$ durch inverse Fouriertransformation eines Signals $\dot{S}_m(v)$ berechnet wird, mit:

$$\tilde{S}_m(v) = \tilde{S}_{0m0}(v) + \tilde{S}_{1m0}(v) + \tilde{S}_{-1m0}(v) \qquad (17),$$

wobei:

$$\tilde{S}_{0m0}(v) = 1/4\big[\tilde{S}_{m0}(v) + \tilde{S}_{m1}(v) + \tilde{S}_{m2}(v) + \tilde{S}_{m3}(v)\big] \qquad (14)$$

$$\tilde{S}_{1m0}(v) = 1/4[\tilde{S}_{m0}(v) - \tilde{S}_{m2}(v) - i(\tilde{S}_{m1}(v) - \tilde{S}_{m3}(v))] \qquad (15)$$

$$\tilde{S}_{-1m0}(v) = 1/4[\tilde{S}_{m0}(v) - \tilde{S}_{m2}(v) + i(\tilde{S}_{m1}(v) - \tilde{S}_{m3}(v))] \qquad (16)$$

die Ausdrücke $\check{S}_{mj}(v)$ sind die zeitlichen Fouriertransformierten der Messsignale $S_{mj}(t)$,
j ist eine ganze Zahl zwischen 0 und 3,
v ist die zeitliche Frequenz.

6. Akustooptisches Abbildungsverfahren nach einem der vorhergehenden Ansprüche, wobei die nicht fokussierte akustische Welle aus einer ebenen akustischen Welle und einer divergenten akustischen Welle ausgewählt ist.

7. Akustooptisches Abbildungsverfahren nach einem der vorhergehenden Ansprüche, wobei die Ausbreitungsrichtungen m der nicht fokussierten akustischen Wellen einen Winkelsektor mit einem Winkel zwischen 30 und 50 Grad abdecken.

8. Akustooptisches Abbildungsverfahren nach einem der vorhergehenden Ansprüche, wobei die Ausbreitungsrichtungen m der nicht fokussierten akustischen Wellen durch einen Winkelabstand zwischen 0,5 Grad und 2 Grad getrennt sind.

9. Akustooptisches Abbildungsverfahren nach einem der vorhergehenden Ansprüche, wobei jeder Messvorgang (150) L-mal wiederholt wird, um L rohe Messsignale $S_{ijl}(t)$ zu erfassen, die jeder Ausbreitungsrichtung m einer nicht-fokussierten akustischen Welle und jeder periodischen räumlichen Amplitudenmodulation j zugeordnet sind, und wobei die L rohen Messsignale $S_{mjl}(t)$ miteinander gemittelt werden, um das Messsignal $S_{mj}(t)$ zu bestimmen, das für den Schritt der räumlichen Demodulation (180) verwendet wird.

10. Akustooptisches Abbildungssystem (1) zum Abbilden eines Beobachtungsbereichs (3) eines Mediums (2), wobei das akustooptische Abbildungssystem aufweist:

   - ein Netz (4) von räumlich gleichmäßig verteilten Ultraschallwandlern ($T_k$),
   - eine Lichtemissionsvorrichtung (8),
   - einen Lichtdetektor (9),
   - eine Steuervorrichtung, die so konfiguriert ist, dass sie über den Lichtdetektor (9) eine Vielzahl von Messsignalen $S_{mj}(t)$ erfasst, die mit räumlich modulierten, nicht fokussierten Schallwellen verbunden sind, die sich jeweils in unterschiedlichen Ausbreitungsrichtungen m ausbreiten,

   wobei die Steuervorrichtung so konfiguriert ist, dass sie:

   - die genannten räumlich modulierten, nicht fokussierten akustischen Wellen nacheinander J-mal in den Beobachtungsbereich (3) aussenden zu lassen, mit jeweils J periodischen räumlichen Amplitudenmodulationen j, die eine räumliche Periode gemäß mindestens einer identischen räumlichen Periodizitätsrichtung (X) haben und einer gegebenen Anzahl P von Wandlern entsprechen, wobei die periodischen räumlichen Amplitudenmodulationen j paarweise räumlich in der Phase verschoben sind,
   - die Lichtemissionsvorrichtung (8) in den Beobachtungsbereich (3) gleichzeitig mit jeder der räumlich modulierten, nicht fokussierten akustischen Wellen mindestens eine einfallende Lichtwelle aussenden lassen, um markierte, räumlich modulierte Lichtwellen zu erzeugen, wobei jede markierte, räumlich modulierte Lichtwelle mindestens eine akustooptische Komponente aufweist, deren Frequenz durch mindestens eine der räumlich modulierten, nicht fokussierten akustischen Wellen verschoben wird,
   - durch das Netz (4) aus Wandlern, für jede markierte räumlich modulierte Lichtwelle ein Messsignal $S_{mj}(t)$ erfassen, das der periodischen räumlichen Amplitudenmodulation j und der nicht fokussierten akustischen Welle entspricht, die sich in der Richtung m ausbreitet,
   - eine räumliche Demodulation durchführen, indem die J Messsignale $S_{mj}(t)$, die den verschiedenen periodischen räumlichen Amplitudenmodulationen entsprechen, für dieselbe Ausbreitungsrichtung m kombiniert werden, um ein demoduliertes Signal $S_m(t)$ zu erhalten, das für die Ausbreitungsrichtung m spezifisch ist,
   - Bestimmen eines Bildes von mindestens einem Teil des Beobachtungsbereichs (3) aus den demodulierten Signalen $S_m(t)$.

11. Akustooptisches Abbildungssystem nach Anspruch 10, bei dem das Netz (4) von Wandlern linear ist und sich entlang der genannten räumlichen Periodizitätsrichtung (X) erstreckt.

12. Akustooptisches Abbildungssystem nach Anspruch 10 oder 11, wobei die periodischen räumlichen Amplitudenmodulationen binäre Funktionen sind, so dass nur bestimmte Ultraschallwandler ($T_k$) für eine gegebene periodischen räumlichen Amplitudenmodulation j aktiviert werden.

13. Akustooptisches Abbildungssystem nach einem der Ansprüche 10 bis 12, wobei J gleich 4 ist, die räumliche Periode einer Anzahl P von Wandlern entspricht, die ein Vielfaches von 4 ist, und die Steuereinrichtung so konfiguriert ist, dass sie die periodischen räumlichen Amplitudenmodulationen j paarweise räumlich in der Phase um eine Phase verschiebt, die einem Viertel der räumlichen Periode entspricht.

14. Akustooptisches Abbildungssystem nach Anspruch 13, wobei die Steuereinrichtung so konfiguriert ist, dass sie das demodulierte Signal $S_m(t)$ durch inverse Fouriertransformation eines Signals $\check{S}_m(\nu)$ berechnet, wobei:

$$\tilde{S}_m(\nu) = \tilde{S}_{0m0}(\nu) + \tilde{S}_{1m0}(\nu) + \tilde{S}_{-1m0}(\nu) \tag{17},$$

wobei:

$$\tilde{S}_{0m0}(\nu) = 1/4\big[\tilde{S}_{m0}(\nu) + \tilde{S}_{m1}(\nu) + \tilde{S}_{m2}(\nu) + \tilde{S}_{m3}(\nu)\big] \tag{14}$$

$$\tilde{S}_{1m0}(\nu) = 1/4\big[\tilde{S}_{m0}(\nu) - \tilde{S}_{m2}(\nu) - i(\tilde{S}_{m1}(\nu) - \tilde{S}_{m3}(\nu))\big] \tag{15}$$

$$\tilde{S}_{-1m0}(\nu) = 1/4\big[\tilde{S}_{m0}(\nu) - \tilde{S}_{m2}(\nu) + i(\tilde{S}_{m1}(\nu) - \tilde{S}_{m3}(\nu))\big] \tag{16}$$

die Ausdrücke $\check{S}_{mj}(\nu)$ sind die zeitlichen Fouriertransformierten der Messsignale $S_{mj}(t)$,
j ist eine ganze Zahl zwischen 0 und 3,
$\nu$ ist die zeitliche Frequenz.

15. Akustooptisches Abbildungssystem nach einem der Ansprüche 10 bis 14, wobei die Steuereinrichtung so konfiguriert ist, dass sie L rohe Messsignale $S_{mjl}(t)$ erfasst, die jeder Ausbreitungsrichtung m einer nicht fokussierten akustischen Welle und jeder periodischen räumlichen Amplitudenmodulation j zugeordnet sind, und wobei die L Messsignale $S_{mjl}(t)$ miteinander gemittelt werden, um das für die räumliche Demodulation verwendete Messsignal $S_{mj}(t)$ zu bestimmen.

**Claims**

1. An acousto-optic imaging method for imaging a region of observation (3) of a medium (2), the method comprising:

   - a measurement step (100) during which a plurality of measurement signals $S_{mj}(t)$ associated with unfocused acoustic waves respectively propagating in various directions of propagation m, are acquired by optical means, said unfocused acoustic waves being respectively emitted in the region of observation (3) by an array (4) of ultrasonic transducers ($T_k$) regularly spatially distributed, said unfocused acoustic waves being emitted, in each direction of propagation m, successively J times with respectively J periodic spatial amplitude modulations j in order to form spatially-modulated unfocused acoustic waves, the periodic spatial amplitude modulations j having an identical spatial period in at least one direction (X) of spatial periodicity, and corresponding to a given number P of transducers, the periodic spatial amplitude modulations j being mutually spatially phase-shifted, the measurement step comprising, for each periodic spatial amplitude modulation j, a plurality of successive measurement operations (150) comprising the following sub-steps:

      o an acoustic emission sub-step in which a spatially-modulated unfocused acoustic wave is emitted with said periodic spatial amplitude modulation j and propagates in a direction of propagation m,
      o a light emission sub-step in which, at the same time as the spatially-modulated unfocused acoustic wave, an incident light wave is emitted in the region of observation (3) in order to generate a spatially-modulated tagged light wave, comprising at least one acousto-optic component respectively shifted in frequency by the spatially-modulated unfocused acoustic wave,

o an acquisition sub-step in which the spatially-modulated tagged light wave is captured and a measurement signal $S_{mj}(t)$ is thus acquired corresponding to the periodic spatial amplitude modulation j and to the unfocused acoustic wave propagating in the direction m,

- a spatial demodulation step, during which the various measurement signals $S_{mj}(t)$ corresponding to the J periodic spatial amplitude modulations are combined for a same direction of propagation m, in order to obtain a demodulated signal $S_m(t)$ specific to the direction of propagation m,
- a processing step (200) during which an image of at least a part of the region of observation (3) is determined using the demodulated signals $S_m(t)$.

2. The acousto-optic imaging method as claimed in claim 1, in which the array (4) of transducers is linear and extends in said direction of spatial periodicity (X).

3. The acousto-optic imaging method as claimed in either of claims 1 and 2, in which said periodic spatial amplitude modulations j are binary functions, such that only certain ultrasonic transducers $(T_k)$ are activated for a given periodic spatial amplitude modulation j.

4. The acousto-optic imaging method as claimed in any one of the preceding claims, in which J is equal to 4, said spatial period corresponds to a number P of transducers being a multiple of 4, and the periodic spatial amplitude modulations j are mutually spatially phase-shifted by a phase corresponding to a quarter of said spatial period.

5. Acousto-optic imaging method as claimed in claim 4, in which, during the spatial demodulation step, the demodulated signal $S_m(t)$ is calculated by inverse Fourier transform of a signal $S_m(v)$, with:

$$\tilde{S}_m(v) = \tilde{S}_{0m0}(v) + \tilde{S}_{1m0}(v) + \tilde{S}_{-1m0}(v) \qquad (17),$$

where:

$$\tilde{S}_{0m0}(v) = 1/4\big[\tilde{S}_{m0}(v) + \tilde{S}_{m1}(v) + \tilde{S}_{m2}(v) + \tilde{S}_{m3}(v)\big] \qquad (14)$$

$$\tilde{S}_{1m0}(v) = 1/4\big[\tilde{S}_{m0}(v) - \tilde{S}_{m2}(v) - i(\tilde{S}_{m1}(v) - \tilde{S}_{m3}(v))\big] \qquad (15)$$

$$\tilde{S}_{-1m0}(v) = 1/4\big[\tilde{S}_{m0}(v) - \tilde{S}_{m2}(v) + i(\tilde{S}_{m1}(v) - \tilde{S}_{m3}(v))\big] \qquad (16)$$

the terms $\tilde{S}_{mj}(v)$ are the time-domain Fourier transforms of the measurement signals $S_{mj}(t)$,
j is an integer in the range between 0 and 3,
$v$ is the time-domain frequency.

6. The acousto-optic imaging method as claimed in any one of the preceding claims, in which the unfocused acoustic wave is chosen from between a plane acoustic wave and a divergent acoustic wave.

7. The acousto-optic imaging method as claimed in any one of the preceding claims, in which the directions of propagation m of the unfocused acoustic waves cover an angular sector with an angle in the range between 30 and 50 degrees.

8. The acousto-optic imaging method as claimed in any one of the preceding claims, in which the directions of propagation m of the unfocused acoustic waves are separated by an angular pitch in the range between 0.5 and 2 degrees.

9. The acousto-optic imaging method as claimed in any one of the preceding claims, in which each measurement operation (150) is repeated L times so as to acquire L raw measurement signals $S_{ijl}(t)$ associated with each direction of propagation m of an unfocused acoustic wave and with each periodic spatial amplitude modulation j, and in which said L raw measurement signals $S_{mjl}(t)$ are averaged together in order to determine the measurement signal $S_{mj}(t)$ used for the spatial demodulation step (180).

10. An acousto-optic imaging System (1) for imaging a region of observation (3) of a medium (2), the acousto-optic imaging system comprising:

- an array (4) of ultrasonic transducers ($T_k$) regularly spatially distributed,
- a light-emitting device (8),
- a light detector (9),
- a control device configured for acquiring, via the light detector (9), a plurality of measurement signals $S_{mj}(t)$ associated with spatially-modulated unfocused acoustic waves respectively propagating in different directions of propagation m,

the control device being configured for:

- causing said spatially-modulated unfocused acoustic waves to be emitted in the region of observation (3) successively J times, with respectively J periodic spatial amplitude modulations j having an identical spatial period in at least one direction of spatial periodicity (X), and corresponding to a given number P of transducers, the periodic spatial amplitude modulations j being mutually spatially phase-shifted,
- causing at least one incident light wave to be emitted by the light-emitting device (8) in the region of observation (3), at the same time as each of said spatially-modulated unfocused acoustic waves, in order to generate spatially-modulated tagged light waves, each spatially-modulated tagged light wave comprising at least one acousto-optic component shifted in frequency by at least one of said spatially-modulated unfocused acoustic waves,
- acquiring by the array (4) of transducers, for each spatially-modulated tagged light wave, a measurement signal $S_{mj}(t)$ corresponding to the periodic spatial amplitude modulation j and to the unfocused acoustic wave propagating in the direction m,
- carrying out a spatial demodulation by combining the J measurement signals $S_{mj}(t)$ corresponding to the various periodic spatial amplitude modulations for a same direction of propagation m, in order to obtain a demodulated signal $S_m(t)$ specific to the direction of propagation m,
- determining an image of at least a part of the region of observation (3) using the demodulated signals $S_m(t)$.

11. The acousto-optic imaging system as claimed in claim 10, in which the array (4) of transducers is linear and extends in said direction of spatial periodicity (X).

12. The acousto-optic imaging system as claimed in either of claims 10 and 11, in which said periodic spatial amplitude modulations j are binary functions, such that only certain ultrasonic transducers ($T_k$) are activated for a given periodic spatial amplitude modulation j.

13. The acousto-optic imaging system as claimed in any one of claims 10 to 12, in which J is equal to 4, said spatial period corresponds to a number P of transducers being a multiple of 4 and the control device is configured for mutually spatially phase shifting said periodic spatial amplitude modulations j by a phase corresponding to a quarter of said spatial period.

14. Acousto-optic imaging system as claimed in claim 13, in which the control device is configured for calculating the demodulated signal $S_m(t)$ by inverse Fourier transform of a signal $\tilde{S}_m(v)$, with:

$$\tilde{S}_m(v) = \tilde{S}_{0m0}(v) + \tilde{S}_{1m0}(v) + \tilde{S}_{-1m0}(v) \qquad (17),$$

where:

$$\tilde{S}_{0m0}(v) = 1/4[\tilde{S}_{m0}(v) + \tilde{S}_{m1}(v) + \tilde{S}_{m2}(v) + \tilde{S}_{m3}(v)] \qquad (14)$$

$$\tilde{S}_{1m0}(v) = 1/4[\tilde{S}_{m0}(v) - \tilde{S}_{m2}(v) - i(\tilde{S}_{m1}(v) - \tilde{S}_{m3}(v))] \qquad (15)$$

$$\tilde{S}_{-1m0}(v) = 1/4\left[\tilde{S}_{m0}(v) - \tilde{S}_{m2}(v) + i(\tilde{S}_{m1}(v) - \tilde{S}_{m3}(v))\right] \qquad (16)$$

the terms $\check{\tilde{S}}_{mj}(v)$ are the time-domain Fourier transforms of the measurement signals $S_{mj}(t)$,
j is an integer in the range between 0 and 3,
$v$ is the time-domain frequency.

15. The acousto-optic imaging system as claimed in any one of claims 10 to 14, in which the control device is configured for acquiring L raw measurement signals $S_{mjl}(t)$ associated with each direction of propagation m of an unfocused acoustic wave and with each periodic spatial amplitude modulation j, and in which said L measurement signals $S_{mjl}(t)$ are averaged together in order to determine the measurement signal $S_{mj}(t)$ used for the spatial demodulation.

FIG. 1

FIG. 2

FIG. 3

FIG. 6

FIG. 4

FIG. 5A

FIG. 5B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016193554 A

**Littérature non-brevet citée dans la description**

- **DE DANIEL S. ELSON ; RUI LI ; CHRISTOPHER DUNSBY ; ROBERT ECKERSLEY ; MENG-XING TANG.** Ultrasound-mediated optical tomography: a review of current methods. *Interface Focus,* 2011, vol. 1, 632-648